# EUROPÄISCHE PATENTSCHRIFT

(11) **EP 4 735 409 B1**
(45) Veröffentlichungstag und Bekanntmachung des Hinweises auf die Patenterteilung: **11.11.2026**
(21) Anmeldenummer: 24737407.7
(22) Anmeldetag: 26.06.2024
(51) Int. Cl.: C07C 29/151, C07C 31/04, B01D 61/42, B01D 61/44

(54) **CO2-NEGATIVE METHANOLSYNTHESE**
CO2-NEGATIVE METHANOL SYNTHESIS
SYNTHÈSE DE MÉTHANOL SANS ÉMISSION DE CO2

(30) Priorität: 28.06.2023 DE 102023206122
(43) Veröffentlichungstag der Anmeldung: 06.05.2026
(73) Patentinhaber: Volkswagen Aktiengesellschaft, 38440 Wolfsburg (DE)
(72) Erfinder: SCHUETZ, Christine, 38440 Wolfsburg (DE); MAAS, Christian, 38440 Wolfsburg (DE); LANGE, Enrica, 38440 Wolfsburg (DE); GROTE, Andreas, 38440 Wolfsburg (DE)
(74) Vertreter: MFG Patentanwälte Meyer-Wildhagen Meggle-Freund Gerhard PartG mbB
(86) Internationale Anmeldenummer: PCT/EP2024/067920
(87) Internationale Veröffentlichungsnummer: WO 2025/003200

(56) Entgegenhaltungen:
- US-A1- 2017 342 004
- "14th International Symposium on Process Systems Engineering", vol. 49, 1 January 2022, ELSEVIER, ISSN: 1570-7946, article GALÁN GUILLERMO ET AL: "Nature vs engineering: Production of methanol from CO2 capture", pages: 271 - 276, XP093203330, DOI: 10.1016/B978-0-323-85159-6.50045-2

## Beschreibung

Die Erfindung betrifft ein Verfahren zur CO₂-negativen Methanolsynthese, eine Anlage zur CO₂-negativen Methanolsynthese sowie eine Verwendung eines Verfahrens und einer Anlage, wie hierin beschrieben.

Die Emission von Kohlendioxid in die Atmosphäre gilt aktuell als wesentlicher Treiber des Klimawandels. Kohlenstoffabscheidungs- und Speichertechniken (Carbon capture and storage - CCS) sind effiziente und effektive Methoden zur Reduzierung der Kohlendioxidemissionen in die Atmosphäre.

Zur Senkung des CO₂-Ausstoßes können zum einen CO₂-negative Emissionstechnologien eingesetzt werden, zum anderen können Technologien zur CO₂-Reduktion in der Atmosphäre bzw. Abgasen eingesetzt werden. Der Bedarf an derartigen Technologien steigt stetig.

CO₂ kann als Ausgangsstoff diverser chemischer Reaktionen, insbesondere zur Herstellung von Basischemikalien und Kraftstoffen (z. B. e-Fuels), dienen. Durch den Einsatz von CO₂ aus der Atmosphäre bzw. aus Abgasen können so Basischemikalien und Kraftstoffe mit CO₂-neutralem bzw. CO₂-negativem Fußabdruck bereitgestellt werden.

Eisaman et al, Clean Technol. 2009, 175-178 beschreibt die energieeffiziente elektrochemische CO₂-Abscheidung aus der Atmosphäre. Die Umsetzung von CO₂ erfolgt dabei mittels Elektrodialyse.

Voskian et al, Energy Environ. Sci., 2019,12, 3530-3547 offenbart eine elektrochemische Vorrichtung für die CO₂-Abscheidung auf der Basis von Chinonen.

US 2017/342004 A1 beschreibt ein Verfahren zur Kraftstoffherstellung aus wässriger Lösung.

Galän et al., Proceedings of the 14th International Symposium on Process Systems Engineering 1. Januar 2022, ISSN:1570-7949, Bd. 49, Seiten 271-276 beschreibt die Produktion von Methanol aus CO₂-Abscheidung.

Aufgabe der vorliegenden Erfindung ist es, ein Verfahren zur CO₂-negativen Methanolsynthese, eine Anlage zur CO₂-negativen Methanolsynthese sowie die Verwendung des Verfahrens bzw. der Anlage bereitzustellen, das bzw. die die oben genannten Nachteile wenigstens teilweise überwinden.

Diese Aufgabe wird durch das erfindungsgemäße Verfahren nach Anspruch 1, die Anlage nach Anspruch 8 bzw. die Verwendung nach Anspruch 13 gelöst.

Gegenstand der vorliegenden Erfindung ist ein Verfahren zur CO₂-negativen Methanolsynthese umfassend:
- mindestens eine Elektrodialyse, und
- mindestens eine Methanolsynthese,
wobei die mindestens eine Elektrodialyse mindestens eine CO₂-haltige Absorberlösung umfasst, wobei die mindestens eine CO₂-haltige Absorberlösung eine aminhaltige CO₂-haltige Absorberlösung ist.

Unter einem CO₂-negativen Verfahren versteht man ein Verfahren, in dem mehr CO₂ generiert als verbraucht wird. Bei einer CO₂-negativen Methanolsynthese versteht man also ein Verfahren zur Herstellung von Methanol, in dem mehr CO₂ gewonnen als verbraucht wird.

Bei einem erfindungsgemäßen Verfahren umfasst die mindestens eine Elektrodialyse eine CO₂-haltige Absorberlösung.

Bei einer Absorberlösung handelt es sich um ein flüssiges Medium, in dem bestimmte Stoffe, wie Gase (z. B. CO₂), absorbiert werden und im Idealfall auch wieder desorbiert werden können. Wie ausgeführt, handelt es sich im vorliegenden Verfahren um eine CO₂-haltige Absorberlösung. Die im Verfahren eingesetzte Absorberlösung kann also CO₂ absorbieren und vorzugsweise auch wieder desorbieren.

Die CO₂-haltige Absorberlösung kann CO₂ in einer Menge im Bereich von 0,1 M bis 2,5 M, vorzugsweise im Bereich von 0,15 M bis 2 M, weiter bevorzugt im Bereich von 0,15 M bis 1,2 M, bezogen auf die Gesamtmenge der Absorberlösung aufnehmen.

Die mindestens eine CO₂-haltige Absorberlösung ist eine aminhaltige CO₂-haltige Absorberlösung.

Eine aminhaltige CO₂-haltige Absorberlösung umfasst somit mindestens ein Amin. Das Amin umfasst vorzugsweise mindestens eine Aminogruppe (primäre Aminogruppe -NH₂, sekundäre Aminogruppe -NHR, tertiäre Aminogruppe -NR₂) und/oder mindestens eine Guanidinogruppe ([CH(NH₂)₃]⁺).

Die Absorberlösung kann ein Amin, oder eine Kombination von mindestens zwei Aminen aufweisen.

Amine können den Vorteil aufweisen, dass sie die Aufnahmekapazität von Gasen (z. B. CO₂) in Absorberlösungen erhöhen können. Amine können die Reaktion von Gasen (z. B. CO₂) mit Wasser zu wasserlöslichen Verbindungen steigern, da die Amin- bzw. Guanidinogruppen der Amine eine schnelle und gesteigerte Aufnahme von Gasen (z. B. CO₂) in Lösungsmittel, wie Wasser, begünstigen können.

Beispiele für aminhaltige Absorberlösungen sind Lösungen von Aminsoäuren. Die Aminosäure kann ausgewählt sein aus der Gruppe bestehend aus Arginin, Alanin, Asparagin, Tyrosin, Isoleucin, Phenylalanin, Cystein, Glycin, Glutamin, Prolin, Histidin, Serin, Leucin, Lysin, Tryptophan, Valin, Methionin und Threonin.

Die Absorberlösung weist einen pH-Wert im Bereich von 8 bis 14 auf. Vorzugsweise weist die Absorberlösung einen pH-Wert im Bereich 10 bis 14 auf. Weiter bevorzugt weist die Absorberlösung einen pH-Wert im Bereich 13 bis 14 auf.

Die Absorberlösung kann alternativ einen pH-Wert im Bereich 8 bis 13 aufweisen, vorzugsweise im Bereich von 9 bis 12, weiter bevorzugt im Bereich 10 bis 11.

Die aminhaltige CO₂-haltige Absorberlösung kann CO₂ in einer Menge im Bereich von 0,5 M bis 2,5 M aufweisen, vorzugsweise im Bereich von 0,8 M bis 2 M, weiter bevorzugt im Bereich von 1 M bis 1,8 M, bezogen auf die Gesamtmenge der Absorberlösung.

Ein erfindungsgemäßes Verfahren umfasst den Schritt der mindestens einen Methanolsynthese. Bei der Methanolsynthese kann es sich um eine direkte Methanolsynthese handeln. Bei der direkten Methanolsynthese wird CO₂ mit H₂, vorzugsweise metallkatalysiert, zu Methanol umgesetzt (Gleichung 1).

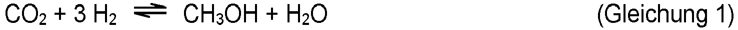

Die Methanolsynthese eines erfindungsgemäßen Verfahrens kann eine reverse Wassergas-Shift-Reaktion umfassen. Bei der Wassergas-Shift-Reaktion wird CO mittels Wasserdampf zu CO₂ und Wasserstoff umgesetzt. Bei der reversen Wassergas-Shift-Reaktion handelt es sich um die Umkehrreaktion. Hier werden CO₂ und Wasserstoff zu CO und Wasser gemäß Gleichung 2 umgesetzt:

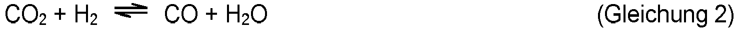

Die reverse Wassergas-Shift-Reaktion findet vorzugsweise bei niedrigen Drücken, beispielsweise bei Drücken um Bereich von 1 bar bis 15 bar, vorzugsweise 1 bis 12 bar, weiter bevorzugt 1 bis 10 bar, statt.

Mittels eines sogenannten Mitteldruckverfahrens kann das CO aus der reversen Wassergas-Shift-Reaktion mit Wasserstoff zu Methanol umgesetzt werden (Gleichung 3).

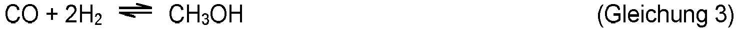

Die Methanolsynthese kann auch eine Kombination aus direkter Methanolsynthese und reverser Wassergas-Shift-Reaktion zusammen mit einem Mitteldruckverfahren sein.

Ein erfindungsgemäßes Verfahren umfasst ferner mindestens eine Elektrodialyse. Bei der Elektrodialyse können Wasserstoff und Sauerstoff gebildet werden. Wie beschrieben, umfasst die Elektrodialyse mindestens eine CO₂-haltige Absorberlösung. Durchläuft die CO₂-haltige Absorberlösung die Elektrodialyse, können so CO₂, Wasserstoff und Sauerstoff generiert werden.

Das CO₂ kann verschiedenen Speichermöglichkeiten, wie z. B. Mineralisierung von CO₂, Gaskartuschen, etc., zugeführt werden. Das gespeicherte CO₂ kann anschließend in diversen Verfahren eingesetzt werden.

Das CO₂ aus der Elektrodialyse kann direkt oder über die Speichermöglichkeit der Methanolsynthese zugeführt werden. Das CO₂ der Methanolsynthese kann dabei komplett der Wassergas-Shift-Reaktion oder komplett der direkten Methanolsynthese zugeführt werden. Alternativ kann das CO₂ der Methanolsynthese zu einem Teil der Wassergas-Shift-Reaktion und zu einem Teil der direkten Methanolsynthese zugeführt werden.

Der Wasserstoff aus der Elektrodialyse kann ebenfalls gespeichert werden oder wird vorzugsweise ebenfalls in der Methanolsynthese eingesetzt. Der Wasserstoff kann dann, sofern vorhanden, der reversen Wassergas-Shift-Reaktion, und/oder direkt der Methanolsynthese, wie dem Mitteldruckverfahren oder der direkten Methanolsynthese zugeführt werden. Überschüssiger Wasserstoff der Methanolsynthese kann abgeführt werden und/oder beispielsweise, falls vorhanden, der reversen Wassergas-Shift-Reaktion zugeführt werden.

Der Wasserstoff, der der Methanolsynthese zugeführt wird, kann komplett der reversen Wassergas-Shift-Reaktion oder komplett der direkten Methanolsynthese zugeführt werden. Alternativ kann ein Teil des Wasserstoffs der reversen Wassergas-Shift-Reaktion und ein Teil der direkten Methanolsynthese zugeführt werden.

Die mindestens eine Elektrodialyse umfasst in einer Ausführungsform mindestens eine CO₂-Wäsche. Die CO₂-Wäsche kann in einem CO₂-Wäscher durchgeführt werden. Bei dem CO₂-Wäscher kann es sich um einen Rieselbettreaktor handeln, dem Luft, Gas, oder eine Gasmischung zugeführt wird. Das Gas bzw. die Gasmischung kann aus einer sogenannten Punktquelle (z. B. eine Gaskartusche) stammen. Dem CO₂-Wäscher wird vorzugsweise ebenfalls die Absorberlösung zugeführt, beispielsweise in reverser Richtung zur Zuführung der Luft oder der Gasmischung. Nach Durchquerung des CO₂-Wäschers kann die Absorberlösung umfassend CO₂ den CO₂-Wäscher verlassen und gleichzeitig ein Luft- bzw. Gasstrom mit vermindertem CO₂-Gehalt den CO₂-Wäscher verlassen.

Die so erhaltene CO₂-haltige Absorberlösung kann anschließend die Elektrodialyse durchlaufen und so das CO₂ wieder freigesetzt werden. Das CO₂ kann schließend, falls vorhanden, in der reversen Wassergas-Shift-Reaktion oder der direkten Methanolsynthese eingesetzt werden.

Die mindestens eine Elektrodialyse kann mindestens einen Membrankontaktor umfassen. Der Membrankontaktor kann alternativ zu einem CO₂-Wäscher vorhanden sein.

Ein Membrankontaktor ist ein Reaktor, der mindestens eine Membran, wie eine Hohlfasermembran, umfasst. Ein Membrankontaktor kann dazu eingesetzt werden, um Gase zu reinigen, bzw. bestimmte Bestandteile von Gasgemischen zu entfernen. Im vorliegenden Verfahren kann der Membrankontaktor dazu verwendet werden, um CO₂ aus der Luft oder einem Gasgemisch zu entfernen. Dabei wird ein Luft- bzw. Gasgemischstrom, umfassend CO₂, dem Membrankontaktor zugeführt und im Gegenstrom wird die Absorberlösung dem Membrankontaktor zugeführt. Die Absorberlösung kann so CO₂ aufnehmen, wodurch der Luft- bzw. Gasstrom CO₂-reduziert, bzw. CO₂-frei ist.

Bei einem erfindungsgemäßen Verfahren können mehrere Elektrodialysen miteinander gekoppelt sein. In Ausführungsformen kann eine oder können mehrere Elektrodialysen eine CO₂-Wäsche umfassen. In alternativen Ausführungsformen kann eine oder können mehrere Elektrodialysen einen oder mehrere Membrankontaktoren umfassen. Es sind auch Ausführungsformen denkbar, in denen eine oder mehrere Elektrodialysen CO₂-Wäschen umfassen und eine oder mehrere Elektrodialysen Membrankontaktoren umfassen.

Ein erfindungsgemäßes Verfahren kann ferner ein Direct Air Capture Verfahren (DAC) umfassen. Bei einem Direct Air Capture Verfahren wird das CO₂ aus der Umgebungsluft gewonnen. Das CO₂ kann in einem geeigneten Abscheideapparat aus der Umgebungsluft abgetrennt werden. Das CO₂ kann anschließend weiterverwendet werden. Durch ein Direct Air Capture Verfahren kann das zur Verfügung stehende CO₂ aufkonzentriert werden. Beispielsweise kann das CO₂ in einem Bereich von ca. 5-85% aufkonzentriert werden und so vorzugsweise aufkonzentriert der Elektrodialyse zugeführt werden.

Das CO₂ in einem erfindungsgemäßen Verfahren kann aus verschiedenen Quellen stammen. Vorzugsweise stammt das CO₂ aus der Atmosphäre. Mit anderen Worten wird das CO₂ vorzugsweise aus der Umgebungsluft gewonnen. Alternativ kann das CO₂ auch aus Abgasen, z.B. von Industrieanlagen, stammen. Bei den Industrieanlagen kann es sich u.a. um Zementwerke, Aluminiumwerke, Kraftwerke, etc. handeln.

Durch die Verwendung von CO₂ aus der Atmosphäre kann der CO₂-Fußabdruck des Methanols eines erfindungsgemäßen Verfahrens gesenkt werden.

Ein weiterer Gegenstand der vorliegenden Erfindung ist eine Anlage zur CO₂-negativen Methanolsynthese umfassend:
- mindestens eine Elektrodialyse-Zelle,
- mindestens einen Methanolsynthese-Reaktor,
wobei die mindestens eine Elektrodialyse mindestens eine CO₂-haltige Absorberlösung umfasst, wobei die mindestens eine CO₂-haltige Absorberlösung eine aminhaltige CO₂-haltige Absorberlösung ist.

Wie bereits in einem erfindungsgemäßen Verfahren umfasst die mindestens eine Elektrodialyse mindestens eine aminhaltige CO₂-haltige Absorberlösung umfassen. Bei der aminhaltigen CO₂-haltigen Absorberlösung kann es sich um eine entsprechende Absorberlösung, wie hierin beschrieben, handeln.

In einer erfindungsgemäßen Anlage kann die mindestens eine Elektrodialyse mindestens einen CO₂-Wäscher umfassen. Bei dem CO₂-Wäscher kann es sich um einen CO₂-Wäscher wie hierin beschrieben handeln.

Alternativ zu einem CO₂-Wäscher kann eine erfindungsgemäße Anlage umfassend eine Elektrodialyse-Zelle mindestens einen Membrankontaktor umfassen. Bei dem mindestens einen Membrankontaktor kann es sich um einen Membrankontaktor, wie hierin beschrieben, handeln.

Eine erfindungsgemäße Anlage kann ferner mindestens eine Direct Air Capture Einheit umfassen. Wie bereits beschrieben ermöglicht die Direct Air Capture Einheit die Versorgung der Anlage mit CO₂, vorzugsweise aus der Umgebungsluft, oder aus Abgasen, z.B. von Industrienanlagen.

In bevorzugten Ausführungsformen stammt das CO₂ aus der Atmosphäre. Vorzugsweise kann so der CO₂-Fußabdruck des Methanols, das in einer erfindungsgemäßen Anlage hergestellt wird, gesenkt werden.

Ferner ist Gegenstand der vorliegenden Erfindung die Verwendung eines Verfahrens, wie hierin beschrieben oder einer Anlage, wie hierin beschrieben, zur Herstellung von Methanol für die Kraftstoffherstellung.

Ein Verfahren, bzw. eine Anlage, wie hierin beschrieben, kann bzw. können auch zur Herstellung von Methanol für chemische Synthesen, als Ausgangsprodukt für chemische Prozesse, als Lösungsmittel, etc. verwendet werden. Weiterhin ist es möglich mit einer Anlage, wie hierin beschrieben, und einem Verfahren, wie hierin beschrieben, weitere organische Basischemikalien herstellen.

Weitere vorteilhafte Ausgestaltungen der Erfindung ergeben sich aus den Unteransprüchen und der folgenden Beschreibung bevorzugter Ausführungsbeispiele der vorliegenden Erfindung.

Ausführungsbeispiele der Erfindung werden nun beispielhaft und unter Bezugnahme auf die beigefügten Zeichnungen beschrieben, in der:
- Fig. 1: eine Übersicht verschiedener Amine,
- Fig. 2: CO₂-Kapazitäten,
- Fig. 3: CO₂-Kapazitäten,
- Fig. 4: die DAC-Effizienz basierend auf dem Gesamtvolumen,
- Fig. 5: ein Fließschema eines exemplarischen Verfahrens,
- Fig. 6: die Desorption von CO₂ aus wässriger Arginin-KOH-Lösung mittels Elektrodialyse,
- Fig. 7: eine Anordnung eines CO₂-Wäschers und einer Elektrodialyse,
- Fig. 8: eine Anordnung eines Membrankontaktors und einer Elektrodialyse, und
- Fig. 9: eine Reihenschaltung mehrerer Elektrodialysen, zeigen.

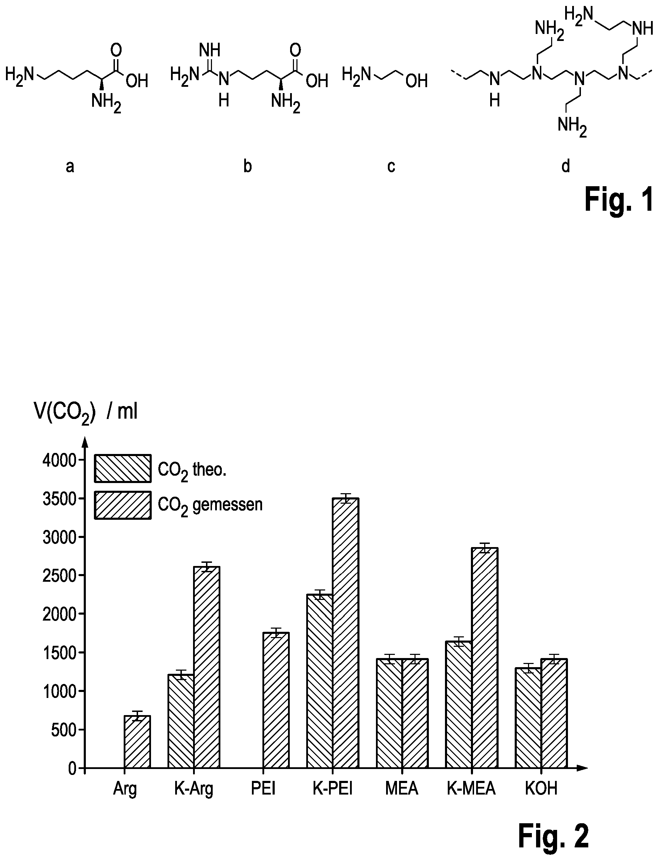

Fig. 1 zeigt eine Übersicht verschiedener Amine. Fig. 1a zeigt L-Lysin. Fig. 1b zeigt L-Arginin. Fig. 1c zeigt Ethanolamin (MEA) und Fig. 1d zeigt Polyethylenimin (PEI).

Fig. 2 zeigt einen Vergleich von pH-basierten theoretisch berechneten und experimentell bestimmten CO₂-Kapazitäten. Die Kapazitäten beziehen sich auf die Desorption nach Sättigung mit reinem CO₂. Die CO₂-Kapazitäten wurden bestimmt mit einer Absorberlösung umfassend L-Arginin. Die Gleichgewichtsreaktion der CO₂-Absorption läuft nach folgenden Gleichungen ab:

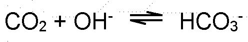

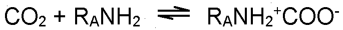

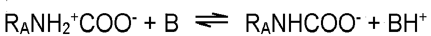

B steht in der Gleichung für einen Protonenakzeptor, z. B. eine Base. Eine beispielhafte Base wäre KOH.

Die theoretische CO₂-Kapazität lässt sich nach folgenden Gleichungen berechnen: $\left.\frac{{dn}_{{OH}^{-}}}{dt}=\frac{{dn}_{{CO}_{2}}}{dt}\right| {\int }_{t = 0}^{t = end}$ ${n}_{OH} = {n}_{{CO}_{2}}$ wobei "n" für Stoffmenge steht.

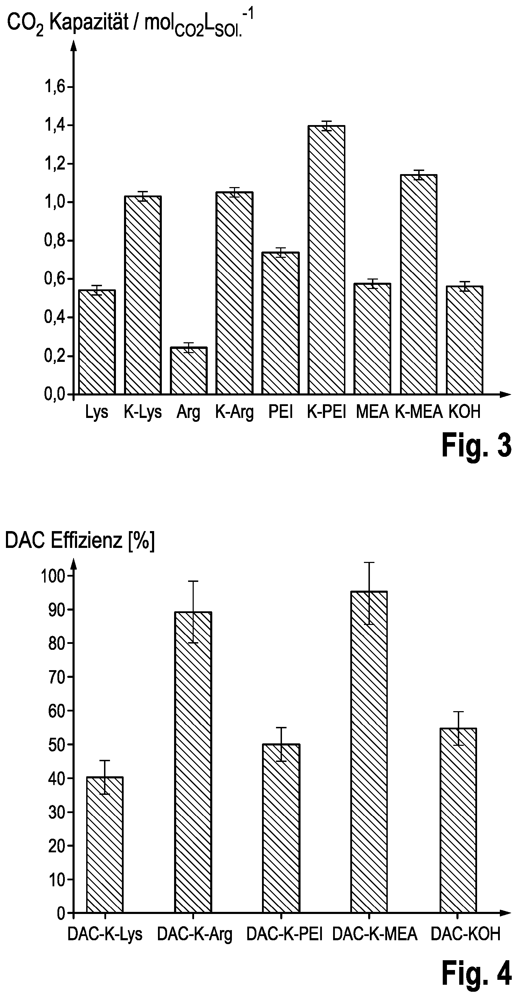

Fig. 3 zeigt die CO₂-Kapazitäten verschiedener Lösungen durch Desorption nach Sättigung der Lösungen mit reinem CO₂. Dabei zeigen die aminbasierten Lösungen die höchsten CO₂-Kapazitäten.

Fig. 4 zeigt die DAC-Effizienz, basierend auf dem Gesamtvolumen, welche die Lösung passiert hat. Die DAC-Effizienz wurde während einer Einwirkung von Druckluft für mindestens 4h bei einer Flussrate von 10 L/min Luft gemessen. Die Berechnungsgrundlage der DAC-Effizienz ist: ${E}_{DAC} = \left({V}_{CO 2 exp .}\right) / = \left({V}_{CO 2 total}\right)$ ${V}_{CO 2 total} = {V}_{Luft total} ⋅ {c}_{CO 2} in Luft$ ${V}_{Luft total} = {t}_{reac .} ⋅ G ,$ wobei G der Luftstrom ist und im DAC-Verfahren 10 L/min betragen hat.

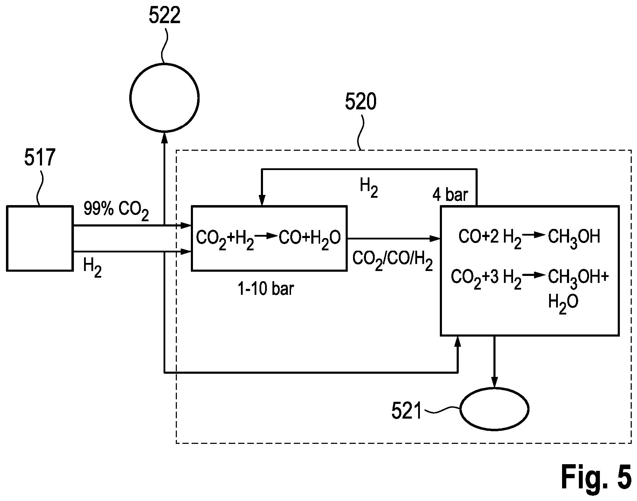

Fig. 5 zeigt ein Fließschema eines exemplarischen erfindungsgemäßen Verfahrens. Zunächst kann mittels Direct Air Capture das CO₂ aus der Umgebungsluft durch Absorption in eine Absorberlösung (z.B. Metallcarbonate, aminhaltig, basisch oder aminosäurehaltig) abgeschieden werden. Anschließend wird mittels Elektrodialyse 517 das CO₂ aus der mit CO₂ angereicherten bzw. CO₂ gesättigten Absorberlösung abgetrennt. Dabei findet ebenfalls eine Elektrolyse des Wassers statt. Sowohl das entstehende H₂ als auch das CO₂ können für die nachfolgende Methanolsynthese 520 verwendet werden. Da weniger CO₂ für die Methanolsynthese 520 benötigt wird als bei der Elektrolyse entsteht, kann das überschüssige CO₂ sequestriert oder anderweitig verwendet werden, wodurch das Methanol CO₂-negativ hergestellt werden kann. Beispielsweise kann das CO₂ durch Mineralisation 522 gespeichert werden. Baut man die Elektrodialyse in Reihe geschaltet auf (mehrere Stacks nebeneinander), kann der Anteil, der sequestriert werden kann, noch erheblich erhöht werden, da die Elektrolyse des Wassers jeweils nur an Kathode und Anode stattfindet. Anschließend erfolgt die Methanolsynthese 520. Ein Teil des Massenstromes von CO₂ wird für die Reverse Wassergas Shift-Reaktion (CO₂ + H₂ 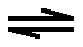 CO + H₂O) genutzt. Das dort erzeugte Synthesegas wird in der nachfolgenden Methanolsynthese 520 (Mitteldruckverfahren: CO + 2H₂ 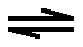 CH₃OH) weiterverwendet. Der bei der Elektrodialyse 517 erzeugte H₂ geht in die Reverse Wassergas Shift-Reaktion, wobei es im dort erzeugten Synthesegas zur nachfolgenden Methanolsynthese (Mitteldruckverfahren) gelangt. Das verbleibende H₂ wird unter Umgehung der Reverse Wassergas-Shift-Reaktion der nachfolgenden (direkten) Methanolsynthese 520 (CO₂ + 3 H₂ 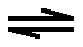 CH₃OH + H₂O) zugeführt. Die Methanolsynthese 520 wird als Mitteldruckverfahren durchgeführt, wobei das überschüssige CO₂ aus dem Mitteldruckverfahren mit dem direkt zugeführten H₂ in der direkten Methanolsynthese reagieren kann. Das ggf. überschüssiges H₂ von der Methanolsynthese 520 kann wieder zur Reverse Wassergas-Shift-Reaktion zurückgeführt werden. Das so entstandene Methanol kann zur Kraftstoffgewinnung (z. B. Gewinnung von e-Fuels) 521 verwendet werden.

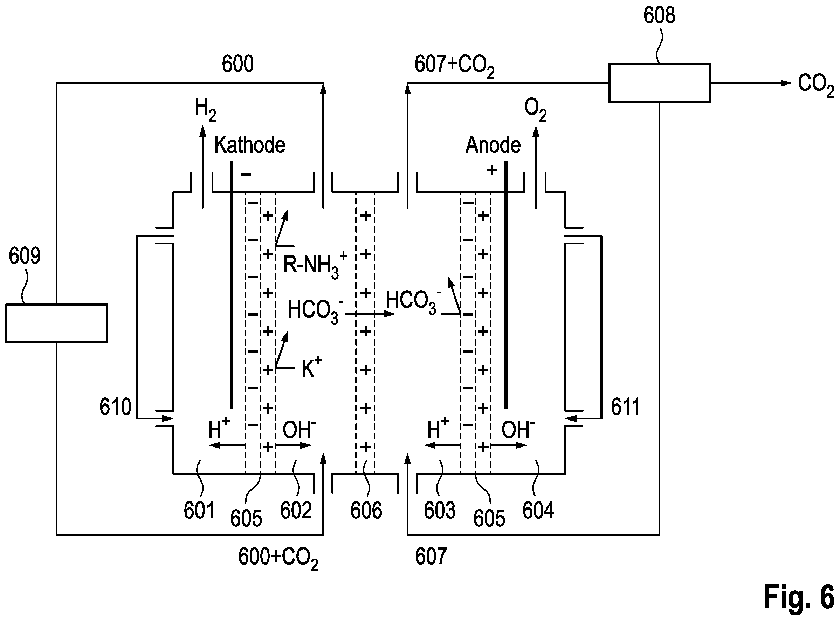

Fig. 6 zeigt die Desorption von CO₂ aus wässriger Arginin-KOH-Lösung (Absorberlösung 600) mittels Elektrodialyse. Eine beispielhafte Elektrodialyse-Zelle besteht aus einer Kathodenkammer 601, einer Diluatkammer 602, einer Konzentratkammer 603 und einer Anodenkammer 604. Die Diluatkammer 602 ist von der Kathodenkammer 601 mittels Bipolarer Membran 605 getrennt. Eine Anionenaustauschermembran 606 trennt die Diluatkammer 602 von der Konzentratkammer 603. Die Konzentratkammer 603 ist von der Anodenkammer 604 mittels Bipolarer Membran 605 getrennt. Während des Elektrodialyseprozesses werden durch Wasserspaltung an der Bipolaren Membran 605 H⁺- und OH⁻-Ionen erzeugt und jeweils in die benachbarte Zellkammer abgegeben. Als Katholyt 610 wurde NaOH eingesetzt. Als Anolyt 611 wurde NaOH eingesetzt.

Die Wasserspaltung erfolgt wie folgt:

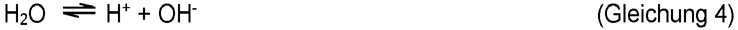

Konkret werden OH⁻-Ionen in die Diluatkammer 602 und H⁺-Ionen in die Konzentratkammer 603 abgegeben.

Die Absorberlösung 600 wird mittels CO₂ Absorption aus der Luft 609 zur mit CO₂ beladenen Arginin-KOH-Lösung (Absorberlösung 600 + CO₂).

Die mit CO₂ beladene Arginin-KOH-Lösung (Absorberlösung 600 + CO₂) wird in die Diluatkammer 602 der Elektrodialysezelle geleitet. Das Anlegen einer elektrischen Potentialdifferenz erzeugt einen elektrochemisch getriebenen Membranprozess, so dass die Hydrogencarbonat-Anionen (HCO₃⁻) aus der Diluatkammer 602 durch eine Anionenaustauschermembran 606 in die Konzentratkammer 603 wandern. Die Weiterwanderung in Richtung Anode wird durch die nachfolgende Bipolarmembran 605 verhindert. In der Konzentratkammer 603 reagieren die Hydrogencarbonat-Anionen mit H⁺ aus Gleichung 4 wie folgt:

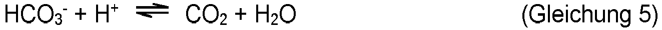

Somit wird HCO₃⁻ in der Diluatkammer 602 reduziert und in der Konzentratkammer 603 aufkonzentriert.

Die R-NH₃⁺ Kationen der beladenen Arginin-KOH-Lösung können nicht ins Konzentrat oder durch die Bipolarmembran 605 in die Kathodenkammer wandern, sondern verbleiben in der Diluatkammer 602 und reagieren mit OH⁻ aus Gleichung 4 wie folgt:

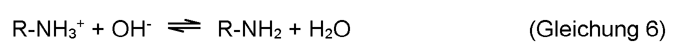

Die Arginin-KOH-Lösung (Absorberlösung 600) ist regeneriert und kann (wieder) einem Wäscher oder einem Membrankontaktor zugeführt werden.

Das CO₂ in der Konzentratkammer 603 wird mittels Verdrängung (pH-Senkung) durch Zuführung eines Aufnahme- und Freisetzungsmediums 607 ausgetrieben. Das Aufnahme- und Freisetzungsmedium 607 ist beispielsweise eine wässrige Lösung einer organischen Säure (zum Beispiel Zitronensäure oder Ascorbinsäure) oder einer anorganischen Säure (zum Beispiel Schwefelsäure oder Diphosphorsäure). Das Aufnahme- und Freisetzungsmedium 607 besitzt vorzugsweise eine sehr hohe elektrische Leitfähigkeit und sollte nicht im elektrischen Gleichspannungsfeld elektrophoretisch transportiert werden.

Während der Elektrodialyse wurden in den Elektrodenkammern H₂ und O₂ als Nebenprodukte freigesetzt. An der Kathode entstand H₂ und an der Anode O₂, die mittels Elektrolytlösung (zum Beispiel NaOH) abgeführt werden können. Unter Wasserabscheidung/CO₂-Freisetzung 608 wurde CO₂ freigesetzt.

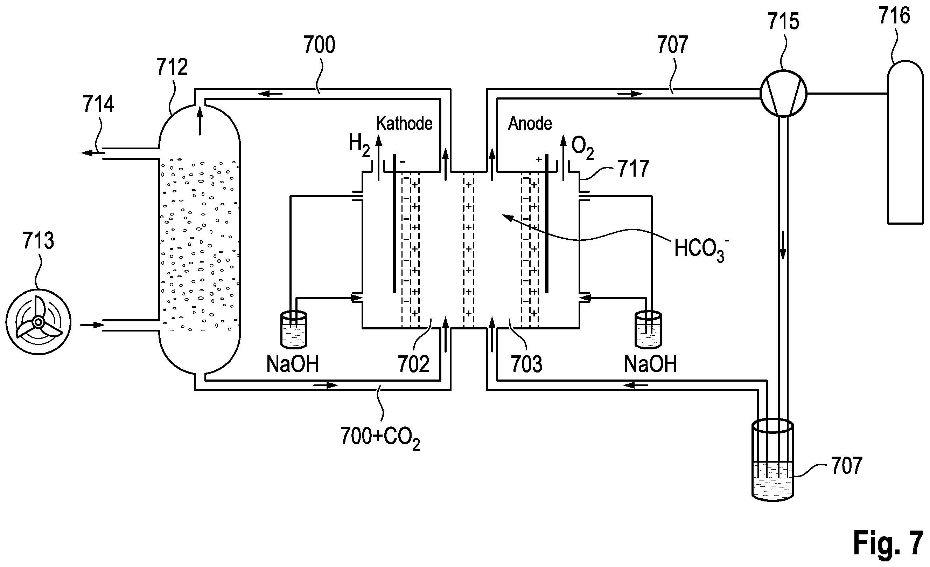

Fig. 7 zeigt eine Anordnung eines CO₂-Wäschers 712 und einer Elektrodialyse. Beispielsweise kann eine derartige Anordnung in einem DAC-Verfahren Anwendung finden. Atmosphärenluft 713 wird in den Wäscher 712 (bevorzugt von unten nach oben) geleitet und die unbeladene Absorberlösung 700 im Gegenstrom (bevorzugt von oben nach unten) zugeführt. CO₂ geht aus der Gasphase in die flüssige Phase nach folgenden Gleichungen 7 und 8 über:

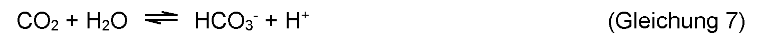

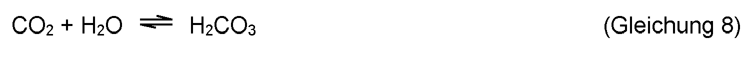

Bei Gleichung 1 handelt es sich um die Hydrolyse und Ionisierung des gelösten CO₂. Gleichung 2 zeigt die Bildung von Kohlensäure.

Außerdem reagiert H⁺ aus Gleichung 1 mit der Amin-Gruppe R-NH₂ des Amins in der erfindungsgemäßen Absorberlösung 700 nach folgender Gleichung 9:

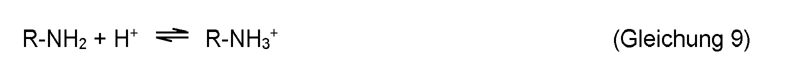

Das gereinigte (CO₂-freie bzw. CO₂-reduzierte) Gas 714 verlässt den Wäscher am Kopf. Die mit CO₂ beladene Absorberlösung 700 wird am Sumpf des Wäschers abgeleitet und in die Elektrodialyse-Zelle 717 gepumpt. In der Elektrodialyse-Zelle 717 kann das CO₂ aus der beladenen Absorberlösung 700 desorbiert werden.

Die Elektrodialyse-Zelle 717 kann der Elektrodialyse-Zelle aus Figur 5 entsprechen.

Das Aufnahme- und Freisetzungsmedium 707 ist beispielsweise eine wässrige Lösung einer organischen Säure (zum Beispiel Zitronensäure oder Ascorbinsäure) oder einer anorganischen Säure (zum Beispiel Schwefelsäure oder Diphosphorsäure) mittels dem das CO₂ wieder freigesetzt wurde. Mittels Entfeuchtung/Verdichtung 715 wird aufkonzentriertes CO₂ 716 gewonnen.

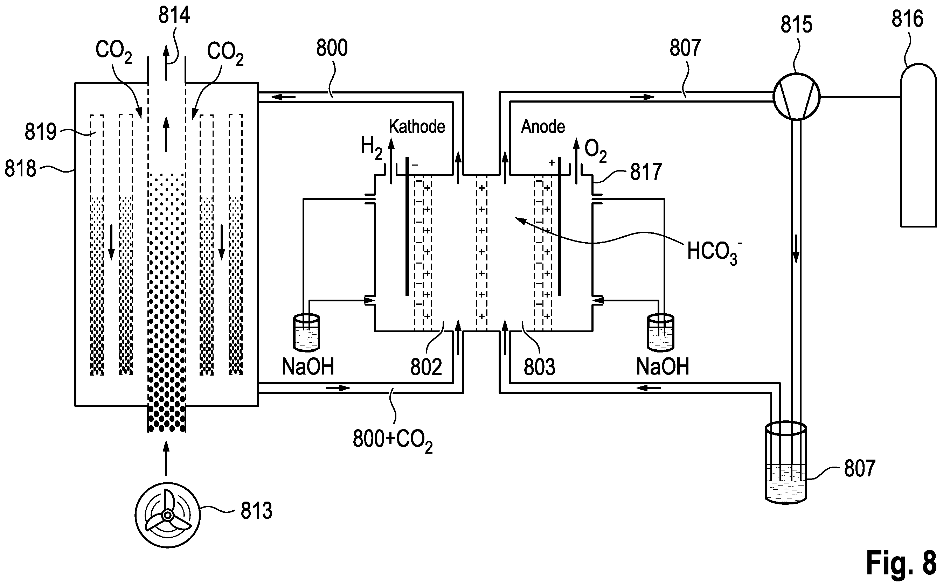

Fig. 8 zeigt eine Anordnung eines Membrankontaktors 818 und einer Elektrodialyse. Beispielsweise kann eine derartige Anordnung in einem DAC-Verfahren Anwendung finden. Durch Verwendung eines Membrankontaktors 818 anstelle eines CO₂-Wäschers kann gegebenenfalls der Wasserverlust während der Absorption verringert werden. Atmosphärenluft 813 wird in den Membrankontaktor 818 (bevorzugt von unten nach oben) geleitet und die unbeladene Absorberlösung 800 im Gegenstrom (bevorzugt von oben nach unten) zugeführt. Der Membrankontaktor 818 weist CO₂-selektive Membranen 819 auf. CO₂ geht aus der Gasphase in die flüssige Phase nach folgenden Gleichungen 7 und 8 über:

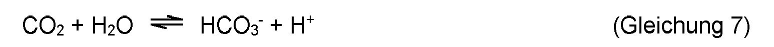

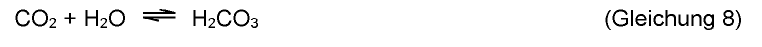

Bei Gleichung 7 handelt es sich um die Hydrolyse und Ionisierung des gelösten CO₂. Gleichung 8 zeigt die Bildung von Kohlensäure.

Außerdem reagiert H⁺ aus Gleichung 7 mit der Amin-Gruppe R-NH₂ des Amins in der erfindungsgemäßen Absorberlösung 800 nach folgender Gleichung 9:

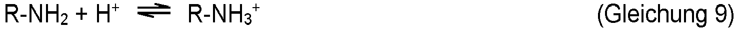

Das gereinigte (CO₂-freie bzw. CO₂-reduzierte) Gas 814 verlässt den Membrankontaktor 818 am Kopf. Die mit CO₂ beladene Absorberlösung 800 wird am Sumpf des Membrankontaktors 818 abgeleitet und in die Elektrodialyse-Zelle gepumpt. In der Elektrodialyse-Zelle 817 kann das CO₂ aus der beladenen Absorberlösung 800 desorbiert werden.

Das Aufnahme- und Freisetzungsmedium 807 ist beispielsweise eine wässrige Lösung einer organischen Säure (zum Beispiel Zitronensäure oder Ascorbinsäure) oder einer anorganischen Säure (zum Beispiel Schwefelsäure oder Diphosphorsäure) mittels dem das CO₂ wieder freigesetzt wurde. Mittels Entfeuchtung/Verdichtung 815 wird aufkonzentriertes CO₂ 816 gewonnen.

Die Elektrodialyse-Zelle 817 kann der Elektrodialyse-Zelle aus Figur 6 entsprechen.

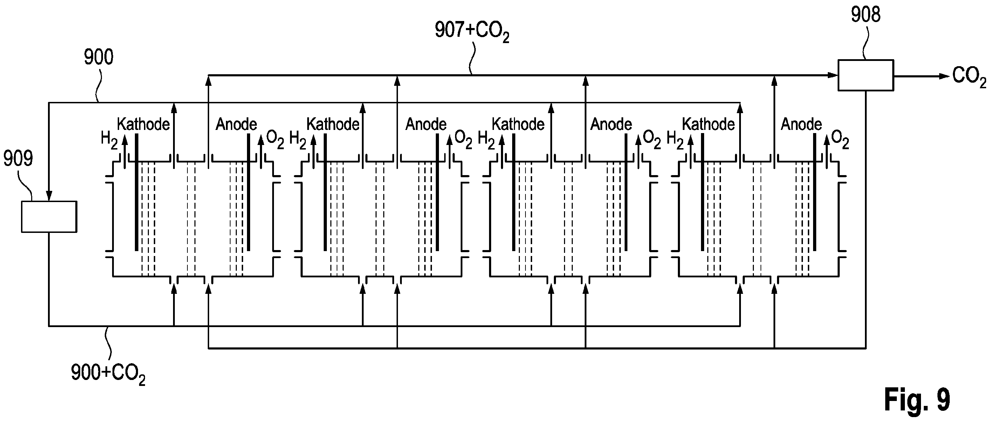

Fig. 9 zeigt eine Reihenschaltung mehrerer Elektrodialyse-Zellen. Die einzelnen Elektrodialyse-Zellen der Reihenschaltung können der Elektrodialyse-Zelle aus Figur 6 entsprechen. Das CO₂ wird aus der Luft absorbiert 909 und die Absorberlösung (hier beispielsweise Arginin-KOH-Lösung) 900 mit CO₂ beladen. Die mit CO₂ beladene Absorberlösung 900 wird den einzelnen Elektrodialyse-Zellen zugeführt. Hier folgt die Freisetzung von H₂ und O₂, sowie die Aufnahme des CO₂ in das Freisetzungsmedium 907. Gleichzeitig wird eine regenerierte Absorberlösung 900 (hier beispielsweise Arginin-KOH-Lösung) erneut der CO₂ Absorption aus der Luft 909 zugeführt. Das Freisetzungsmedium 907 + CO₂ wird einer Wasserabscheidung/CO₂-Freisetzung 908 zugeführt, wodurch das CO₂ gewonnen wird und das Freisetzungsmedium 907 wieder erneut den Elektrodialyse-Zellen zugeführt wird.

Durch die Reihenschaltung mehrerer Elektrodialyse-Zellen kann eine beliebig große Menge CO₂ und auch der Gase H₂ und O₂ generiert werden.

### Bezugszeichenliste

- 600, 700, 800, 900: Absorberlösung
- 601: Kathodenkammer
- 602: Diluatkammer
- 603: Konzentratkammer
- 604: Anodenkammer
- 605: Bipolare Membran
- 606: Anionenaustauschermembran
- 607, 707, 807, 907: Aufnahme- und Freisetzungsmedium
- 608, 908: Wasserabscheidung/CO₂-Freisetzung
- 609: Luft
- 610: Katholyt
- 611: Anolyt
- 712: CO₂-Wäscher
- 713, 813: Atmosphärenluft
- 714, 814: gereinigtes (CO₂-freies bzw. CO₂-reduziertes) Gas
- 715, 815: Entfeuchtung/Verdichtung
- 716, 816: aufkonzentriertes CO₂
- 517, 717, 817: Elektrodialyse-Zelle
- 818: Membrankontaktor
- 819: CO₂-selektive Membran
- 520: Methanolsynthese
- 521: Kraftstoffgewinnung
- 522: Mineralisation

## Patentansprüche

1. Verfahren zur CO₂-negativen Methanolsynthese umfassend:
- mindestens eine Elektrodialyse, und
- mindestens eine Methanolsynthese,
wobei die mindestens eine Elektrodialyse mindestens eine CO₂-haltige Absorberlösung umfasst,
wobei die mindestens eine CO₂-haltige Absorberlösung eine aminhaltige CO₂-haltige Absorberlösung ist.

2. Verfahren nach Anspruch 1, wobei die Methanolsynthese eine direkte Methanolsynthese ist.

3. Verfahren nach Anspruch 1 oder Anspruch 2, wobei die Methanolsynthese eine reverse Wassergas-Shift-Reaktion umfasst.

4. Verfahren nach mindestens einem der Ansprüche 1 bis 3, wobei die mindestens eine Elektrodialyse mindestens eine CO₂-Wäsche umfasst.

5. Verfahren nach mindestens einem der Ansprüche 1 bis 4, wobei die Elektrodialyse mindestens einen Membrankontaktor umfasst.

6. Verfahren nach mindestens einem der Ansprüche 1 bis 5, wobei das Verfahren ferner ein Direct Air Capture Verfahren umfasst.

7. Verfahren nach mindestens einem der Ansprüche 1 bis 6, wobei das CO₂ aus der Atmosphäre stammt.

8. Anlage zur CO₂-negativen Methanolsynthese umfassend:
- mindestens eine Elektrodialyse-Zelle,
- mindestens einen Methanolsynthese-Reaktor,
wobei die mindestens eine Elektrodialyse mindestens eine CO₂-haltige Absorberlösung umfasst,
wobei die mindestens eine CO₂-haltige Absorberlösung eine aminhaltige CO2-haltige Absorberlösung ist.

9. Anlage nach Anspruch 8, wobei die mindestens eine Elektrodialyse mindestens einen CO₂-Wäscher umfasst.

10. Anlage nach mindestens einem der Ansprüche 8 bis 9, wobei die mindestens eine Elektrodialyse mindestens einen Membrankontaktor umfasst.

11. Anlage nach mindestens einem der Ansprüche 8 bis 10, wobei die Anlage ferner eine Direct Air Capture Einheit umfasst.

12. Anlage nach mindestens einem der Ansprüche 8 bis 11, wobei das CO₂ aus der Atmosphäre stammt.

13. Verwendung eines Verfahrens nach mindestens einem der Ansprüche 1 bis 7, oder einer Anlage nach mindestens einem der Ansprüche 8 bis 12 zur Herstellung von Methanol für die Kraftstoffherstellung.

## Claims

1. Method of CO₂-negative methanol synthesis comprising:
- at least one electrodialysis, and
- at least one methanol synthesis,
wherein the at least one electrodialysis comprises at least one CO₂-containing absorber solution,
wherein the at least one CO₂-containing absorber solution is an amine-containing CO₂-containing absorber solution.

2. Method according to Claim 1, wherein the methanol synthesis is a direct methanol synthesis.

3. Method according to Claim 1 or Claim 2, wherein the methanol synthesis comprises a reverse water-gas shift reaction.

4. Method according to at least one of Claims 1 to 3, wherein the at least one electrodialysis comprises at least one CO₂ scrub.

5. Method according to at least one of Claims 1 to 4, wherein the electrodialysis comprises at least one membrane contactor.

6. Method according to at least one of Claims 1 to 5, wherein the method further comprises a direct air capture method.

7. Method according to at least one of Claims 1 to 6, wherein the CO₂ originates from the atmosphere.

8. Plant for CO₂-negative methanol synthesis comprising:
- at least one electrodialysis cell,
- at least one methanol synthesis reactor,
wherein the at least one electrodialysis comprises at least one CO₂-containing absorber solution,
wherein the at least one CO₂-containing absorber solution is an amine-containing CO₂-containing absorber solution.

9. Plant according to Claim 8, wherein the at least one electrodialysis comprises at least one CO₂ scrubber.

10. Plant according to at least one of Claims 8 to 9, wherein the at least one electrodialysis comprises at least one membrane contactor.

11. Plant according to at least one of Claims 8 to 10, wherein the plant further comprises a direct air capture unit.

12. Plant according to at least one of Claims 8 to 11, wherein the CO₂ originates from the atmosphere.

13. Use of a method according to at least one of Claims 1 to 7, or of a plant according to at least one of Claims 8 to 12, for production of methanol for fuel production.

## Revendications

1. Procédé de synthèse de méthanol à bilan CO₂ négatif, comprenant :
- au moins une électrodialyse, et
- au moins une synthèse de méthanol,
dans lequel ladite au moins une électrodialyse comprend au moins une solution absorbante contenant du CO₂,
dans lequel ladite au moins une solution absorbante contenant du CO₂ est une solution absorbante contenant une amine et du CO₂.

2. Procédé selon la revendication 1, dans lequel la synthèse de méthanol est une synthèse directe de méthanol.

3. Procédé selon la revendication 1 ou la revendication 2, dans lequel la synthèse de méthanol comprend une réaction inverse de conversion du gaz à l'eau.

4. Procédé selon au moins l'une des revendications 1 à 3, dans lequel ladite au moins une électrodialyse comprend au moins un lavage du CO₂.

5. Procédé selon au moins l'une des revendications 1 à 4, dans lequel l'électrodialyse comprend au moins un contacteur à membrane.

6. Procédé selon au moins l'une des revendications 1 à 5, dans lequel le procédé comprend en outre un procédé de captage direct dans l'air.

7. Procédé selon au moins l'une des revendications 1 à 6, dans lequel le CO₂ provient de l'atmosphère.

8. Installation de synthèse de méthanol à bilan CO₂ négatif, comprenant :
- au moins une cellule d'électrodialyse,
- au moins un réacteur de synthèse de méthanol,
dans lequel ladite au moins une électrodialyse comprend au moins une solution absorbante contenant du CO₂,
dans lequel ladite au moins une solution absorbante contenant du CO₂ est une solution absorbante contenant une amine et du CO₂.

9. Installation selon la revendication 8, dans laquelle ladite au moins une électrodialyse comprend au moins un laveur de CO₂.

10. Installation selon au moins l'une des revendications 8 à 9, dans laquelle ladite au moins une électrodialyse comprend au moins un contacteur à membrane.

11. Installation selon au moins l'une des revendications 8 à 10, dans laquelle l'installation comprend en outre une unité de captage direct dans l'air.

12. Installation selon au moins l'une des revendications 8 à 11, dans laquelle le CO₂ provient de l'atmosphère.

13. Utilisation d'un procédé selon au moins l'une des revendications 1 à 7 ou d'une installation selon au moins l'une des revendications 8 à 12 pour produire du méthanol destiné à la fabrication de carburant.
